# FASCICULE DE BREVET EUROPEEN

(11) **EP 0 321 354 B1**
(45) Date de publication et mention de la délivrance du brevet: **04.08.1993**
(21) Numéro de dépôt: 88403215.2
(22) Date de dépôt: 16.12.1988
(51) Int. Cl.: B01D 17/032, G01N 33/48, G01N 1/16

(54) **Dispositif assurant l'extraction d'une phase liquide distincte**
Vorrichtung zur Extrahierung einer bestimmten Flüssigkeitsphase
Device assuring the extraction of a distinct liquid phase

(30) Priorité: 17.12.1987 FR 8717633
(43) Date de publication de la demande: 21.06.1989
(73) Titulaire: ROUSSEL-UCLAF, 75007 Paris (FR)
(72) Inventeur: Benet, Daniel, F-93250 Villemomble (FR)
(74) Mandataire: Bourgouin, André

(56) Documents cités:
- EP-A- 0 363 485
- FR-A- 2 561 776
- US-A- 4 420 254
- US-A- 4 577 514
- PATENT ABSTRACTS OF JAPAN, vol. 9, no. 262 (P-398)[1985], 19 octobre 1985, page 117 P 398; & JP-A-60 111 941 (NIPPON TECTRON K.K.) 18-06-1985
- PATENT ABSTRACTS OF JAPAN, vol. 7, no. 152 (P-208)[1297], 5 juillet 1983, page 99 P 208; & JP-A-58 63 852 (HITACHI SEISAKUSHO K.K.) 15-04-1983

## Description

La présente invention a pour objet un dispositif assurant l'extraction d'une phase liquide distincte. Plus particulièrement, elle a pour objet un dispositif assurant l'extraction sélective d'une des phases constituant par exemple les liquides biologiques. Un de ces liquides est notamment le sang.

Dans la demande de brevet européen EP. 0 363 485 non publiée au moment du dépôt prioritaire de la présente demande est décrit une méthode et un dispositif de recueillement du sang.

Cependant ce dispositif prévoit la présence d'un séparateur physique entre les différentes phases du liquide ce que ne prévoit pas le dispositif de la présente demande.

Or, on connaît bien l'importance que représente l'examen des différentes phases du sang, aussi bien dans la recherche que dans les différentes analyses que l'on est amené à faire au cours d'études ou encore dans de différentes préparations.

La séparation sélective de la phase constituée par du plasma sanguin de la phase sanguine globulaire présente un intérêt particulier, de plus il est très important de pouvoir séparer ces deux phases de telle manière que la séparation en question soit faite totalement et d'une manière automatique.

Or dans la recherche ainsi que dans les industries et notamment dans l'industrie pharmaceutique, on a besoin d'appareils susceptibles d'effectuer de nombreuses telles opérations d'une manière exacte et automatique et selon un programme d'ordinateur défini au préalable.

De ce fait, il y a donc de nombreux appareils utilisés dans ce domaine.

Néanmoins, le problème de l'extraction sélective optimale et automatique d'une des phases distinctes par leurs opacités différentes, telle que celle de la phase constituée par du plasma sanguin de la phase sanguine globulaire, n'a toujours pas été résolu de façon satisfaisante surtout quand il s'agit d'appareillages complexes devant fonctionner d'une manière plus ou moins automatique.

En effet d'une manière générale, l'extraction désirée d'une phase n'était pas soumise à un automatisme, qui aurait commandé exactement le commencement et l'arrêt d'extraction de la phase au moment propice et avec l'exactitude voulue. La plupart du temps, les manipulations ayant trait à l'extraction recherchée devaient être commandées ou effectuées manuellement, d'où une perte de temps non négligeable et le risque que l'extraction voulue soit incomplète ou au contraire ne s'arrête pas à temps et que commence alors l'extraction d'une autre phase non souhaitée.

Or, la demanderesse a mis au point le dispositif, objet de l'invention, qui comble cette lacune et permet, de ce fait, d'effectuer l'extraction optimale d'une phase et que cette opération soit commandée automatiquement.

Le dispositif de l'invention permet en outre l'automatisation de la séparation des phases d'opacités différentes quels que soient le volume total de l'échantillon à traiter et la proportion relative des phases en présence.

Le dispositif objet de l'invention permettant dans un système multiphase l'extraction sélective des phases se distinguant par leur différence d'opacité, contenues dans un récipient, le fonctionnement de ce dispositif pouvant être commandé automatiquement, est caractérisé en ce qu'il comporte :
- un étrier mobile par rapport audit récipient contenant les phases d'opacités différentes,
- un moyen associé à l'étrier, sensible au changement d'opacité des phases présentes dans le récipient,
- et un moyen assurant l'extraction recherchée, solidaire de l'étrier.

Le dispositif, objet de l'invention, permettant notamment dans un système multiphase l'extraction sélective des phases se distinguant par leur différence d'opacité, contenues dans un récipient, le fonctionnement de ce dispositif pouvant être commandé automatiquement, est caractérisé en ce qu'il comporte :
- un étrier ① mobile par rapport audit récipient ② contenant notamment des phases d'opacités différentes ③ et ④ ,
- un moyen ⑤ associé à l'étrier ① , sensible au changement d'opacité des phases présentes dans le récipient ② ,
- et un moyen ⑥ assurant l'extraction recherchée, solidaire de l'étrier ① .

Le dispositif objet de l'invention permettant, particulièrement dans un système multiphase, l'extraction sélective des phases se distinguant par leur différence d'opacité, contenues dans un récipient, le fonctionnement de ce dispositif pouvant être commandé automatiquement comprenant :
- un étrier mobile par rapport audit récipient contenant les phases d'opacité différente,
- un moyen associé à l'étrier, sensible au changement d'opacité des phases présentes dans le récipient,
- un moyen assurant l'extraction recherchée, solidaire de l'étrier, est caractérisé en ce que le déplacement et notamment l'arrêt de l'étrier et/ou l'extraction sont commandés par les mesures effectuées par ledit moyen sensible au changement d'opacité.

Dans un mode préféré d'utilisation du dispositif on extrait sélectivement l'une des phases d'un système comprenant deux phases.

Le dispositif de l'invention peut encore être caractérisé en ce que :
- l'étrier ① est exécuté en forme de la lettre grecque d'imprimerie Π ou de la lettre d'imprimerie U inversée, qu'il peut enjamber le récipient ② et qu'il peut se déplacer dans le sens de l'axe longitudinal dudit récipient ②;
- le moyen ⑤ , sensible au changement d'opacité des phases présentes dans le récipient ② , est un senseur optique qui est disposé vers les deux extrémités des branches ⑧ de l'étrier ① ;
- le moyen ⑥ , assurant l'extraction recherchée, est un moyen agissant par aspiration, en forme de pipette et placé dans l'axe du récipient ② ;
- l'extrémité ⑨ du moyen ⑥ en forme de pipette est disposée légèrement en retrait par rapport à la zone ⑦ contrôlée par le moyen ⑤ sensible au changement d'opacité des phases.
- le récipient ② est un récipient utilisé couramment lors de centrifugation tel qu'une éprouvette.

Le procédé d'extraction sélective d'une des phases distinctes par leurs opacités différentes et contenues dans un récipient, également objet de l'invention, est caractérisé en ce que :
- un étrier ① , porteur d'un moyen ⑤ sensible au changement d'opacité et d'un moyen ⑥ assurant l'extraction recherchée, se déplace le long de l'axe dudit récipient ② ;
- en faisant déplacer l'étrier ①, on fait entrer le moyen ⑥ dans le récipient ② puis le fait pénétrer dans une phase ③ contenue dans ledit récipient ② ;
- ladite phase ③ est extraite à l'aide du moyen ⑥ ;
- l'extraction se prolongeant jusqu'à ce que le moyen ⑤ sensible au changement d'opacité des phases, contenues dans le récipient ② ,détecte une phase ayant une opacité différente et commande l'arrêt d'extraction, l'ensemble des opérations pouvant être dirigé automatiquement.

L'invention a également pour objet l'application du dispositif de l'invention à l'extraction du plasma sanguin, après que celui-ci ait été séparé, par exemple par centrifugation, de la phase sanguine globulaire.

L'application du procédé de l'invention à l'extraction du plasma sanguin une fois celui-ci séparé de la phase sanguine globulaire fait aussi l'objet de l'invention.

L'utilisation du dispositif selon l'invention dans un appareillage quelconque fait également l'objet de l'invention.

Le dispositif objet de l'invention peut encore être illustré par les modes d'exécution suivants :
- le récipient ② , sous forme d'une pipette, doit être transparent et peut être échangé aisément, afin d'autoriser l'extraction de différentes phases sans que l'on encoure le risque de contamination ;
- le déplacement de l'étrier ① est effectué par exemple au moyen d'un vérin hydraulique, pneumatique ou électrique, ou tout autre moyen adéquat ;
- l'extraction d'une phase est effectuée par exemple par une pompe créant le vide ;
- le senseur optique peut être un dispositif couramment utilisé pour mesurer habituellement l'opacité ou détecter le changement d'opacité.

Le dispositif de l'invention peut être utilisé en une unité seule, ou bien plusieurs unités identiques peuvent être mises en batteries ; de cette manière, il est possible d'effectuer plusieurs extractions simultanément.

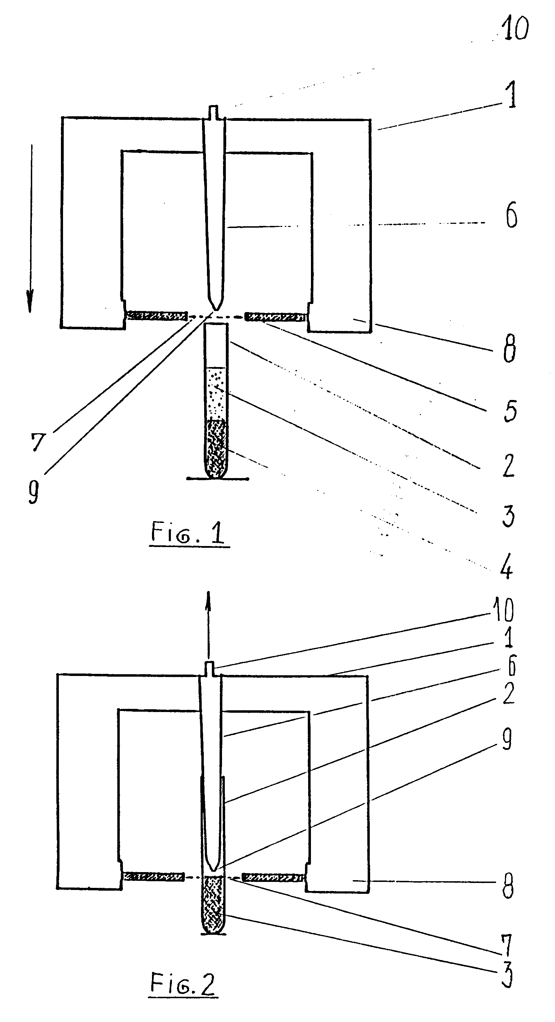

Le dispositif, objet de l'invention, est illustré par les dessins des figures 1 et 2.

Sur ces dessins :
① représente l'étrier en forme de la lettre grecque d'imprimerie Π ou de la lettre latine d'imprimerie U inversée ;
② le récipient contenant des phases distinctes par leurs opacités différentes ;
③ la phase dotée d'une faible opacité ;
④ la phase dotée d'une forte opacité ;
⑤ le moyen sensible au changement d'opacité tel qu'un senseur optique ;
⑥ le moyen assurant l'extraction recherchée, tel qu'une pipette à aspirer ;
⑦ la zone contrôlée par le moyen ⑤ ;
⑧ les extrémités de l'étrier ① ;
⑨ l'extrémité inférieure du moyen ⑥ sous forme d'une pipette ;
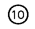 le conduit relié à un dispositif d'aspiration et d'évacuation de la phase extraite.

La figure 1 représente le dispositif de l'invention au repos. C'est-à-dire que la séparation des phases ③ et ④ de différentes opacités n'a pas été encore commandée et l'étrier ① se trouve en position haute, dite de repos.

La figure 2 représente le dispositif de l'invention en sa position basse - dite de la fin de l'extraction de la phase ③ - du plasma sanguin par exemple. Le senseur optique ⑤ ayant dans la zone ⑦ qu'il contrôle, rencontré la phase ④ d'une opacité différente, a donné l'ordre d'arrêter le déplacement de l'étrier ① et permis d'arrêter l'extraction de la phase ③ .

Le fonctionnement du dispositif de l'invention peut être décrit comme suit :
Une fois les phases ③ et ④ de différentes opacités obtenues, par exemple par centrifugation, on commande le déplacement de l'étrier ① porteur de la pipette ⑥ . Celui-ci se faisant, la pipette ⑥ pénètre dans le récipient ② , puis entre en contact avec la phase ③ d'une opacité faible qui est à extraire.

L'ordre d'extraction de cette phase est donné alors et elle commence. Au fur et à mesure que la phase ③ est extraite, par exemple par aspiration, l'étrier ① se déplace afin que la pipette ⑥ reste dans ladite phase ③ . Ce mouvement est maintenu tant que la zone ⑦ contrôlée par le senseur optique ne rencontre pas la limite de la phase ④ d'une opacité différente que celle de la phase ③ .

Au moment où le senseur optique ⑤ constate, dans la zone ⑦ , qu'il contrôle, la présence de la phase ④ , comme par exemple la phase globulaire sanguine, ledit senseur optique ⑤ commande l'arrêt du déplacement de l'étrier ① et l'arrêt de l'extraction de la phase ③ , dans le cas présent du plasma sanguin.

Du fait qu'à ce moment précis, l'extrémité inférieur de la pipette ⑥ , se trouve légèrement en retrait de la zone ⑦ contrôlée par le senseur optique ⑤ et se trouve encore dans ladite phase ③ du plasma sanguin, le présent dispositif assure une extraction optimale de cette phase, sans que la phase ④ , la phase globulaire sanguine soit entrainée par l'aspiration et mélangée à la phase ③ déjà extraite.

Les traitements ultérieurs des phases ③ et ④ ainsi séparées sont assurés par d'autres dispositifs non décrits ici.

Il est évident que le dispositif de l'invention peut être utilement intégré, seul ou en plusieurs unités dans les dispositifs utilisés dans ce domaine, tels que les ensembles d'analyses, de recherche ou de préparation automatisés.

Il va de soi que le dispositif de l'invention ne saurait être limité aux modes d'exécution décrits et illustrés, dont il englobe toutes les variantes.

## Revendications

1. Dispositif permettant dans un système multiphase l'extraction sélective des phases se distinguant par leur différence d'opacité,
contenues dans un récipient, le fonctionnement de ce dispositif pouvant être commandé automatiquement, comportant:
- un étrier mobile par rapport audit récipient contenant les phases d'opacités différentes,
- un moyen à deux branches associé à l'étrier, sensible au changement d'opacité des phases présentes dans le récipient,
- et un moyen assurant l'extraction recherchée, solidaire de l'étrier.

2. Dispositif selon la revendication 1, caractérisé en ce que le déplacement et notamment l'arrêt de l'étrier et/ou l'extraction sont commandés par les mesures effectuées par ledit moyen sensible au changement d'opacité.

3. Dispositif suivant l'une quelconque des revendications 1 ou 2, caractérisé en ce que l'étrier ① est exécuté en forme de la lettre grecque d'imprimerie Π ou de la lettre latine d'imprimerie U inversée, qu'il peut enjamber le récipient ② et qu'il peut se déplacer dans le sens de l'axe longitudinal dudit récipient ② .

4. Dispositif suivant l'une quelconque des revendications 1 à 3, caractérisé en ce que le moyen 5 présent dans le récipient ② est un senseur optique qui est disposé vers les deux extrémités des branches ⑧ de l'étrier ① .

5. Dispositif suivant l'une quelconque des revendications 1 à 4, caractérisé en ce que le moyen ⑥ assurant l'extraction recherchée est un moyen agissant par aspiration en forme de pipette et placé dans l'axe du récipient ② .

6. Dispositif suivant l'une quelconque des revendications 1 à 5, caractérisé en ce que l'extrémité ⑨ du moyen ⑥ en forme de pipette est disposée en retrait par rapport à la zone ⑦ contrôlée par le moyen ⑤ sensible au changement d'opacité des phases.

7. Dispositif suivant l'une quelconque des revendications 1 à 6, caractérisé en ce que le récipient ② est un récipient utilisé couramment lors de centrifugation tel qu'une éprouvette.

8. Procédé d'extraction sélective d'une des phases ③ et ④ distinctes par leurs opacités différentes contenues dans un récipient ② , comportant les étapes:
- un étrier ① , porteur d'un moyen ⑤ à deux branches sensible au changement d'opacité et d'un moyen ⑥ assurant l'extraction recherchée, se déplace le long de l'axe dudit récipient ② ,
- en faisant déplacer l'étrier 1, on fait entrer le moyen ⑥ dans le récipient ② puis le fait pénétrer dans une phase ③ contenu dans ledit récépient ② ;
- ladite phase ③ est extraite à l'aide du moyen ⑥ ;
- l'extraction se prolongeant jusqu'à ce que le moyen ⑤ sensible au changement d'opacité des phases contenues dans le récipient ② détecte une phase ayant une opacité différente et commande l'arrêt d'extraction l'ensemble des opérations pouvant être dirigé automatiquement.

9. Application du dispositif suivant l'une quelconque des revendications 1 à 7, caractérisée en ce qu'il est utilisé pour l'extraction du plasma sanguin une fois celui-ci séparé de la phase sanguine globulaire.

10. Application du procédé selon la revendication 8, à l'extraction du plasma sanguin une fois celui-ci séparé de la phase sanguine globulaire.

11. Utilisation du dispositif selon l'une quelconque des revendications 1 à 7, dans un appareillage quelconque.

## Claims

1. Device permitting the selective extraction, from a multiphase system, of phases which are distinguished by their difference in opacity,
contained in a receptacle, the operation of this device being able to be automatically controlled, comprising:
- a stirrup movable relative to the said receptable containing the phases of different opacity,
- a means with two arms attached to the stirrup, sensitive to the change in opacity of the phases present in the receptacle,
- and a means assuring the desired extraction, integral with the stirrup.

2. Device according to claim 1, characterized in that the displacement and in particular the stopping of the stirrup and/or the extraction are controlled by measures carried out by the said means sensitive to the change in opacity.

3. Device according to any one of claims 1 or 2, characterized in that the stirrup (1) is made in the form of the printed Greek letter Π or the inverted printed Roman letter U, that it can straddle the receptable (2) and that it can move in the direction of the longitudinal axis of the said receptacle (2).

4. Device according to any one of claims 1 to 3, characterized in that the means (5) present in the receptacle (2) is an optical sensor which is positioned towards the two ends of the arms (8) of the stirrup (1).

5. Device according to any one of claims 1 to 4, characterized in that the means (6) assuring the desired extraction is a means which acts by suction in the form of a pipette and placed in a perpendicular fashion inside the receptacle (2).

6. Device according to any one of claims 1 to 5, characterized in that the end (9) of the means (6) in the form of a pipette is placed away from the area (7) controlled by the means (5) sensitive to the change in opacity of the phases.

7. Device according to any one of claims 1 to 6, characterized in that the receptacle (2) is a receptacle commonly used during centrifuging such as a test tube.

8. Process for selective extraction of one of the phases (3) and (4) distinguished by their different opacities contained in a receptacle (2), comprising the (following) stages:
- a stirrup (1), which supports a means (5) with two arms sensitive to the change in opacity and a means (6) assuring the desired extraction, moves along the axis of the said receptacle (2),
- by moving the stirrup (1), the means (6) enters the receptacle (2) then passes through a phase (3) contained in the said receptacle (2);
- the said phase (3) is extracted using the means (6);
- the extraction continues until the means (5) sensitive to the change in opacity of the phases contained in the receptacle (2) detects a phase having a different opacity and stops the extraction, all the operations being able to be automatically controlled.

9. Use of the device according to any one of claims 1 to 7, characterized in that it is used for the extraction of blood plasma once this has been separated from the hemocyte phase.

10. Use of the process according to claim 8, for the extraction of blood plasma once this has been separated from the hemocyte phase.

11. Use of the device according to any one of claims 1 to 7, in any apparatus.

## Patentansprüche

1. Vorrichtung zur selektiven Extrahierung von Phasen in einem Multi-Phasen-System, wobei sich die Phasen durch ihre unterschiedliche Opazität unterscheiden und sich in einem Behälter befinden, und der Betrieb dieser Vorrichtung automatisch gesteuert werden kann, umfassend:
- einen Bügel, der bezüglich dieses Behälters beweglich ist, der die Phasen mit unterschiedlicher Opazität enthält,
- eine Einrichtung an zwei Beinen, die dem Bügel zugeordnet sind, die auf eine Änderung der Opazität der sich in dem Behälter befindenden Phasen empfindlich ist,
- und eine Einrichtung für die gesuchte Extrahierung, die fest mit dem Bügel verbunden ist.

2. Vorrichtung nach Anspruch 1,
dadurch gekennzeichnet,
daß das Verschieben und insbesondere das Anhalten des Bügels und/oder der Extrahierung durch die Messungen gesteuert werden, die durch diese Einrichtung ausgeführt werden, die auf eine Änderung der Opazität empfindlich ist.

3. Vorrichtung nach einem der Ansprüche 1 oder 2,
dadurch gekennzeichnet,
daß der Bügel 1 in einer Form des griechischen Druckbuchstabens Π oder des umgekehrten lateinischen Druckbuchstabens U ausgeführt ist, daß er den Behälter 2 überspannen kann und daß er sich in die Richtung der Längsachse dieses Behälters 2 verschieben kann.

4. Vorrichtung nach einem der Ansprüche 1-3,
dadurch gekennzeichnet,
daß die Einrichtung 5, die in den Behälter 2 hinein wirkt, ein optischer Sensor ist, der auf den beiden Enden der Beine 8 des Bügels 1 angeordnet ist.

5. Vorrichtung nach einem der Ansprüche 1-4,
dadurch gekennzeichnet,
daß die Einrichtung 6 für die gesuchte Extrahierung eine Einrichtung ist, die zum Ansaugen wirkt und in Form einer Pipette ausgebildet ist und in der Achse des Behälters 2 angeordnet ist.

6. Vorrichtung nach einem der Ansprüche 1-5,
dadurch gekennzeichnet,
daß das Ende 9 der Einrichtung 6 in Form einer Pipette etwas zurückgezogen bezüglich des Bereichs 7 angeordnet ist, der durch die Einrichtung 5 kontrolliert wird, die auf eine Änderung der Opazität der Phasen empfindlich ist.

7. Vorrichtung nach einem der Ansprüche 1-6,
dadurch gekennzeichnet,
daß der Behälter 2 ein Behälter ist, der üblicherweise beim Zentrifugieren verwendet wird, so wie ein Reagenzglas.

8. Verfahren zum selektiven Extrahieren einer von den Phasen 3 und 4, die sich durch ihre unterschiedlichen Opazitäten unterscheiden und in einem Behälter 2 enthalten sind, umfassend die Schritte
- ein Bügel 1, der eine Einrichtung 5 an zwei Beinen trägt, die auf eine Änderung der Opazität empfindlich ist, und der eine Einrichtung 6 zum Sicherstellen der gesuchten Extrahierung trägt, verschiebt sich längs der Achse dieses Behälters 2,
- beim Verschieben des Bügels 1 tritt die Einrichtung 6 in den Behälter 2 und taucht dann in eine Phase 3, die in diesem Behälter 2 enthalten ist,
- diese Phase 3 wird mittels der Einrichtung 6 extrahiert,
- die Extrahierung wird fortgesetzt, bis daß die Einrichtung 5, die auf eine Änderung der Opazität der Phasen, die in dem Behälter 2 enthalten sind, empfindlich ist, eine Phase mit einer unterschiedlichen Opazität wahrnimmt und den Befehl zum Beenden der Extrahierung abgibt, wobei die Gesamtheit der Betriebsvorgänge automatisch gesteuert werden kann.

9. Verwendung der Vorrichtung nach einem der Ansprüche 1-7, dadurch gekennzeichnet, daß sie für die Extrahierung von Blutplasma verwendet wird, sobald es von der Phase der Blutkörperchen getrennt ist.

10. Verwendung des Verfahrens nach Anspruch 8 zur Extrahierung von Blutplasma, sobald es von der Phase der Blutkörperchen getrennt ist.

11. Verwendung der Vorrichtung nach einem der Ansprüche 1-7 in irgendeiner Apparatur.
